# EUROPEAN PATENT SPECIFICATION

(11) **EP 1 714 625 B1**
(45) Date of publication and mention of the grant of the patent: **23.05.2012**
(21) Application number: 05075920.8
(22) Date of filing: 19.04.2005
(51) Int. Cl.: A61F 5/44

(54) **A tap for drainage of a bag such as a urine collection bag**
Hahn zur Entleerung eines Beutel wie eines Urinsammelbeutels
Un robinet pour le drainage d'un sac tel qu'un sac de rassemblement d'urine

(43) Date of publication of application: 25.10.2006
(73) Proprietor: Unomedical A/S, 3460 Birkerød (DK)
(72) Inventor: Hansen, Trygve Kalf, 4040 Jyllinge (DK); Einarsson, Per, 4600 Køge (DK)
(74) Representative: Dragsted, Helle Rude

(56) References cited:
- GB-A- 2 118 685
- GB-A- 2 166 222
- US-B1- 6 709 420
- US-B1- 6 722 624

## Description

The present invention relates to a tap for drainage of a urine collection bag, comprising a housing having a first and second flow passage and a substantially cylindrical inner space and a housing bottom; a rotatable valve member, which is rotatably mounted in the housing, is provided with a flow passage, and may be rotated in the housing between a closed valve position and an open valve position where flow communication between the first and second flow passages is provided.

When a urine bag is attached to the leg for daytime use, it is usual to provide a closure valve functioning as a tap for draining the urine bag by activating the valve tap.

A tap of this kind is known from GB-A-2 166 222. This tap discloses a valve member with a lever arm integrally from thereon. This valve member is snap-mounted to the valve housing and rotatable between a closed position, where the arm is parallel to the flow passages of the valve housing and an open position, where the arm is rotated to a position orthogonal to the flow passages as the arm is moved towards a stop on the outside of the housing.

A similar tap of this kind is known from e.g. US patent no. 6,722,624 or WO-A-91/03217. In a tap of this kind, a relative angular motion between the valve member and the arm is provided so that the arm is moved between two parallel positions, i.e. an angular movement of 180°, for rotating the valve member 90° for opening and closing the valve. Hereby, the lever arm need not project unduly in either open or closed positions of the tap. A similar tap is also known from GB-A-2 118 685.

By providing a tap with the lever arm in a parallel position in both valve positions, the risk of accidental operation of the valve is somewhat reduced. However, there is still a substantial risk that the tap lever arm is inadvertently moved away from its open or closed position. The valve may be even just slightly opened if the lever arm is unintentionally moved as a result of the person's activities during the day, or at night, as the lever arm may be pressed against the person's body when the person turns over in his sleep.

The object of the invention is to provide a tap for a urine collection bag of the initially mentioned kind where unintentional movement of the tap is prevented.

This object is achieved by a tap of the above-mentioned kind, wherein the inner space of the housing is provided with first rotation limit stop means which cooperate with second rotation limit stop means on the valve member for limiting the angular displacement of the valve member in the housing.

Conveniently, the valve member in a tap according to the present invention is prevented from being rotated outside the limitation stops. Since the limitation stops are provided internally, and the limitation of angular movement of the valve member is not exposed to the exterior whereby the stops are protected from outside impacts.

Preferably, the first and second rotation stop limiters comprise at least one stop provided in the bottom of the housing cooperating with a stop member provided on the valve member for limiting the angular displacement of the valve member in the housing.

Preferably, the stop member includes two stop heads radially spaced apart at 180°. Hereby, a compact design of the valve member may be provided. Accordingly, two stops are preferably provided with approx. 180° displacement of each other on the bottom of the housing.

Preferably, the stops are protruding upwards from the bottom surface in the axial direction. However, it is realised that alternatively, the angular limitation stops could be grooves in the housing bottom surface and the rotation stop limitation means on the valve member are one or more downwardly protruding portions, e.g. taps extending into the grooves on the bottom. Another alternative could be to provide a stop limit member protruding upwards from the bottom of the housing and cooperates with two stops on the valve member.

In a preferred embodiment, a particularly compact design is provided, where the valve member is formed with a substantially cylindrical valve body with the stop member at the end thereof, and that said cylindrical valve member is inserted in the cylindrical inner space of the housing.

In a preferred embodiment of the invention, the at least one stop is provided with a notch extending radially from the outer side of the stop, and that the valve member is provided with at least one retention notch extending radially on the inner side of the cylindrical valve body for cooperating with the notch on the stop of the housing bottom. Furthermore, the valve body is preferably provided with a first and second retention notch for retaining the valve member in an open and closed position - respectively - in the housing. Hereby, an accidental movement of the lever arm is further prevented as the rotation valve member is retained in its two active positions, i.e. the open and closed position.

In a preferred embodiment, the two retention notches are provided with a radial distance of less than 90°, preferably approximately 80°. Hereby, the cooperating notches of the housing and the valve member have just passed each other when the limitation stops of each of the valve components abut each other.

Preferably, the notch is radially outwardly protruding from the stop. As an alternative to notches protruding out of the valve body wall, the retention notches may be inwardly radially protruding dent in the valve body.

In the following, the invention is described in more detail under reference to the accompanying drawings, in which:
- Fig. 1: shows a cross-section of a tap according to the invention;
- Fig. 2: is a top view of the valve housing of a tap according to the invention;
- Fig. 3: is a bottom view of the valve member of a tap according to the invention;
- Fig. 4: shows a tap according to the invention in an open position;
- Fig. 5: shows the tap in a closed position;
- Fig. 6 and 7: are perspective views of the valve plug member of a tap according to the invention; and
- Figs. 8 and 9: are schematic views of manufacturing techniques for moulding the valve plug member.

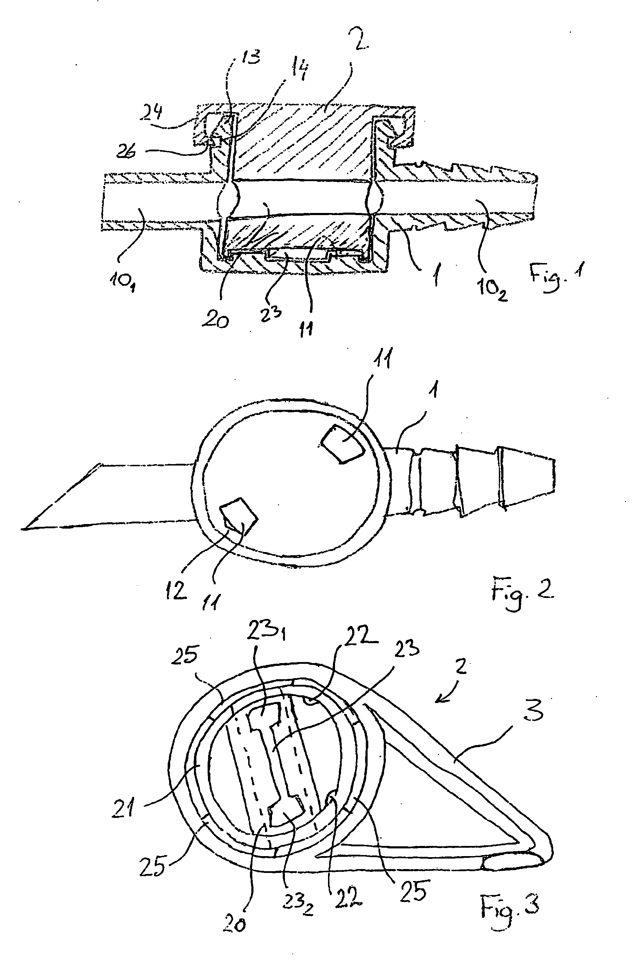

As shown in the figures 1 to 3, the tap for a urine collection bag includes a valve housing 1 with a flow inlet 10₂ and outlet 10₁ cooperating with a valve member 2, which is rotatable in the housing 1 so that a flow passage 20 is aligned with the flow inlet 10₂ and outlet 10₁ of the housing 1 in an open position and where the flow passage 10 is substantially 90° off-set from the housing flow passages 10 in a closed position. The valve member 2 includes an arm 3 for rotating the valve plug member 2 between the open and closed positions.

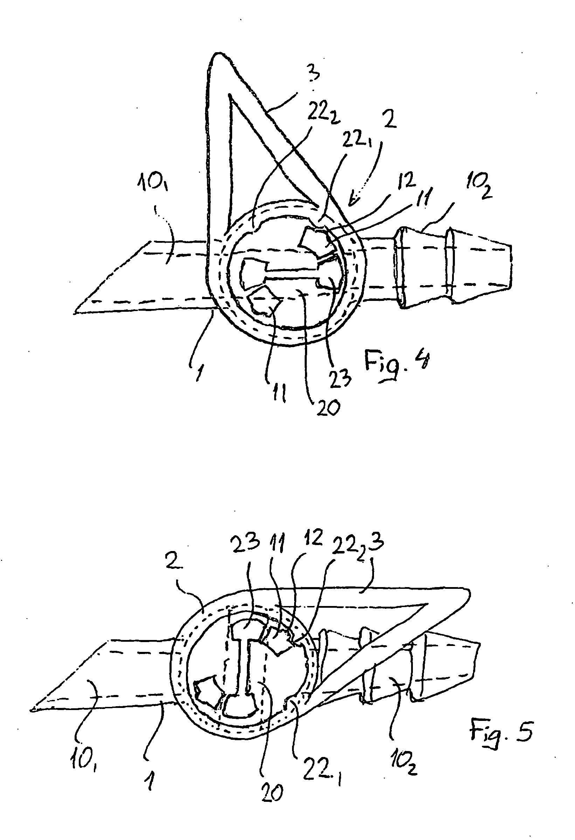

To control the angular movement of the valve member 2 in the valve housing 1, the inner space of the valve housing 1 is cylindrical and the valve plug member 2 is formed correspondingly. Two upwardly protruding stops 11 are formed in the bottom of the valve housing 1 at an angular distance of approx. 180° from each other and with a certain distance from the periphery of the circular bottom surface of the housing. At least one of the stops 11 is provided with a small notch 12, which is provided on the radial outermost side of the stop 11. The valve plug 2 is provided with a cylindrical body 21 with a wall thickness which is smaller than the distance from the notch 12 to the cylindrical inner sidewall of the housing 1 so that the valve member body 21 is allowed to pass the notch 12. The inner side of the cylindrical body 21 is provided with two, radially inwardly facing notches 22 in the end section of the cylindrical body 21. The end section is also provided with a stop member 23 including two stop heads 23₁, 23₂ radially spaced apart at 180°, which are provided for engagement with the stops 11 of the valve housing 1. The two notches 22 are placed with an angular displacement of less than 90°, e.g. approximately 80°, so that when the valve member 2 is rotated near to one stop 11, the notch 12 pass a first notch 22₁ just before the stop member 23 abuts the stop 11, whereby the valve member 2 is retained in this stop position. Similarly, the valve plug member 2 may be retained in a second stop position at the second notch 22₂. The direction of the flow passage 20 of the valve plug member 2 is coordinated with the positions of the stops 23 and the notches 22, so that the first stop is an open position, where the flow passage 20 is aligned with the flow system 10 of the housing I (see fig. 4), and the second position is a closed position, where the flow passage 20 is generally orthogonal to the flow system 10 of the housing 1 (see fig 5).

By the cooperating stops 11, 23 and notches 12, 22, the valve plug member 2 is retained in its two operative positions, as shown in figures 4 and 5. This eliminates the risk of leakage and accidental opening of the tap of the urine collection bag.

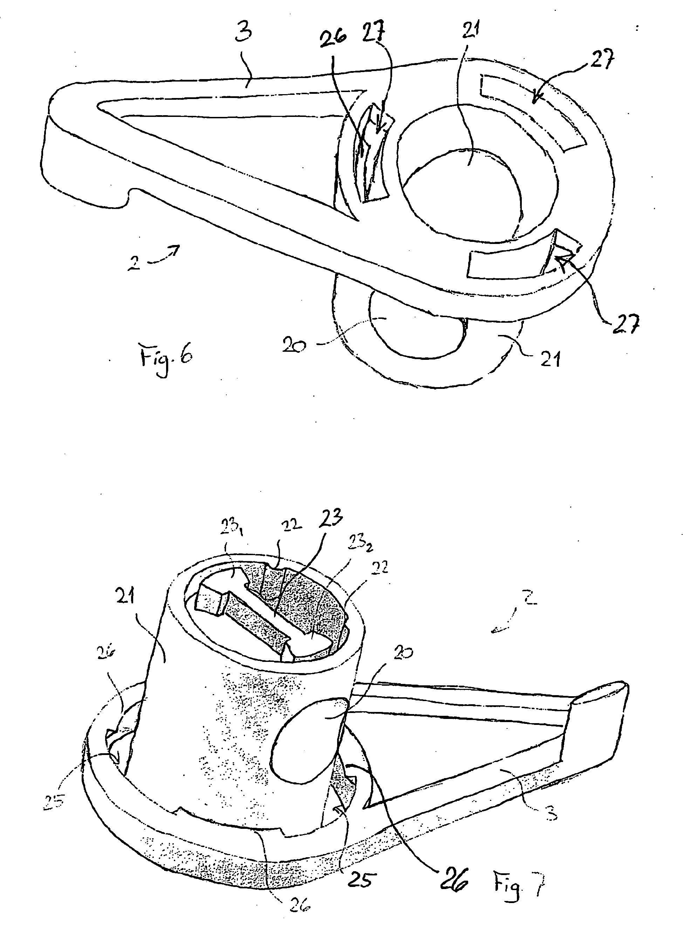

A preferred embodiment of a valve member 2 is shown in figures 6 and 7. An arm 3 for activating the tap is integrally formed in the valve member 2. With reference to fig. 1, the valve member 2 is provided with an interrupted annular collar 24, which cooperates with an annular end section 13 of the housing 1, where the collar 24 has an inwardly protruding portion 26 and is retained in an annular groove 14 of the housing 1 by a snap-engagement. Hereby, the valve member 2 is secured to the housing 1 in a rotatable manner.

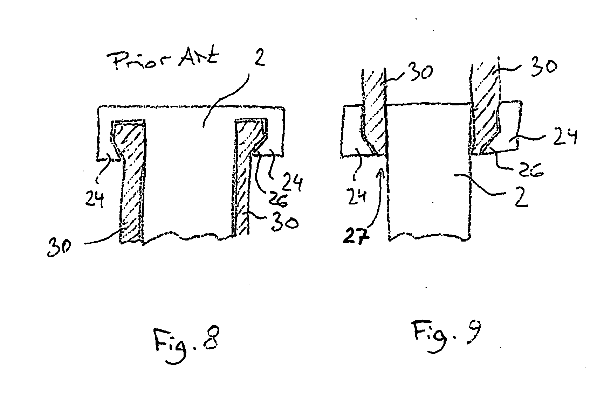

The moulding of the valve member 2 is somewhat difficult, as the moulding of the annular collar 24 in the known snap-engagement tap designs requires a collapsible mould core 30. This mould core 30 extends from below to form the cavity for the collar 24 and the annular, inwardly protruding flange 26 (see fig. 8). To deform, the mould core 30 must be collapsed, which results in a slow moulding process and extraordinary expensive moulding tools. Instead, it is realised that a multiple of retention flanges 26 may be provided instead of one annular flange 26 as shown in fig. 8. Hereby, open spaces 25 are provided in between the flange portions 26 as shown in figs. 3, 6, 7 and 9. As shown in fig. 9, this means that the mould core 30 may extend from above, which makes it easy and quicker to deform the mould.

The invention is described with reference to a preferred embodiment. However, it is realised that other variants of the invention may be provided without departing from the scope of the invention as defined in the accompanying claims.

## Claims

1. A tap for drainage of a urine collection bag, comprising
a housing (1) having
a first and second flow passage (101, 102) and a substantially cylindrical inner space and a housing bottom;
a rotatable valve member (2), which is rotatably mounted in the housing, is provided with a flow passage (20), and may be rotated in the housing (1) between a closed valve position and an open valve position where flow communication between the first and second flow passages (101, 102) is provided;
the inner space of the housing (1) is provided with first rotation limit stop means (11) which cooperate with second rotation limit stop means (23) on the valve member (2) for limiting the angular displacement of the valve member (2) in the housing (1), **characterised in that** at least one stop means (11) is provided with a notch (12) extending radially from the outer side of the stop means (11), and that the valve member (2) is provided with two retention notches (22₁, 22₂) extending radially on the inner side of the cylindrical valve body (21) placed with angular displacement of less than 90° for cooperating with the notch (12) on the stop means (11) of the housing bottom, so that notch (22₁) passes notch (12) in the open position and notch (22₂) passes notch (12) in the closed position just before the first and second rotation limit stop means abut each other.

2. A tap according to claim 1, wherein the first and second rotation limit stop means comprise at least one stop means (11) provided on the bottom of the housing (1) cooperating with a stop member (23) provided on the valve member (2) for limiting the angular displacement of the valve member (2) in the housing (1).

3. A tap according to claim 2, wherein the stop member (23) includes two stop heads (231; 232) radially spaced apart at 180°.

4. A tap according to claim 2 or 3, wherein two stop means (11) are provided with approx. 180° displacement of each other on the bottom of the housing (1).

5. A tap according to any of claims 1 to 4, wherein the stop means (11) of the first rotation limit stop means are protruding upwards from the bottom surface in the axial direction.

6. A tap according to any of the preceding claims, wherein the valve member (2) is formed with a substantially cylindrical valve body (21) with the stop member (23) at the end thereof, and that said cylindrical valve member (2) is inserted in the cylindrical inner space of the housing (1).

7. A tap according to any of the preceding claims, wherein the retention notches (22) are inwardly radially protruding dent in the valve body (21).

8. A tap according to any of the preceding claims, wherein the notch (12) is radially outwardly protruding from the stop means (11).

## Patentansprüche

1. Hahn für die Entleerung eines Urinsammelbeutels, enthaltend
ein Gehäuse (1), das einen ersten und einen zweiten Durchflussdurchgang (101, 102) und einen im wesentlichen zylindrischen Innenraum und einen Gehäuseboden hat;
und ein drehbares Ventilelement (2), das in dem Gehäuse drehbar angebracht und mit einem Durchflussdurchgang (20) versehen ist und in dem Gehäuse (1) zwischen einer geschlossenen Ventilstellung und einer geöffneten Ventilstellung gedreht werden kann, in der eine Durchflussverbindung zwischen dem ersten und dem zweiten Durchflussdurchgang (101, 102) besteht;
wobei der Innenraum des Gehäuses (1) mit ersten Drehanschlagsbegrenzungseinrichtungen (11) versehen ist, die mit zweiten Drehanschlagsbegrenzungseinrichtungen (23) an dem Ventilelement (2) zusammenwirken, um die Winkelverstellung des Ventilelementes (2) in dem Gehäuse (1) zu begrenzen, **dadurch gekennzeichnet, dass** wenigstens eine Anschlagseinrichtung (11) mit einem Vorsprung (12) versehen ist, der sich radial von der Außenseite der Anschlagseinrichtung (11) erstreckt, und dass das Ventilelement (2) mit zwei Rückhaltevorsprüngen (22₁, 22₂) versehen ist, die sich radial auf der Innenseite des zylindrischen Ventilkörpers (21) erstrecken, der mit einer Winkelverstellung von weniger als 90 für das Zusammenwirken mit dem Vorsprung (12) an der Anschlagseinrichtung (11) des Gehäusebodens angeordnet ist, so dass der Vorsprung (22₁) den Vorsprung (12) in der geöffneten Stellung passiert und der Vorsprung (22₂) den Vorsprung (12) in der geschlossenen Stellung passiert, unmittelbar bevor die ersten und die zweiten Drehbegrenzungsanschlagseinrichtungen miteinander in Anlage gelangen.

2. Hahn nach Anspruch 1, bei dem die ersten und die zweiten Drehbegrenzungsanschlagseinrichtungen wenigstens eine Anschlagseinrichtung (11) enthalten, die auf dem Boden des Gehäuses (1) vorgesehen ist und mit einem Anschlagselement (23) zusammenwirkt, das an dem Ventilelement (2) vorgesehen ist, um die Winkelverstellung des Ventilelementes (2) in dem Gehäuse (1) zu begrenzen.

3. Hahn nach Anspruch 2, bei dem das Anschlagselement (23) zwei Anschlagsköpfe (231; 232) enthält, die radial um 180° beabstandet sind.

4. Hahn nach Anspruch 2 oder 3, bei dem zwei Anschlagseinrichtungen (11) mit etwa 180° Verschiebung zueinander auf dem Boden des Gehäuses (1) vorgesehen sind.

5. Hahn nach einem der Ansprüche 1 bis 4, bei dem die Anschlagseinrichtung (11) der ersten Drehbegrenzungsanschlagseinrichtungen nach oben von der Bodenfläche in der axialen Richtung hervorragt.

6. Hahn nach einem der vorhergehenden Ansprüche, bei dem das Ventilelement (2) mit einem im wesentlichen zylindrischen Ventilkörper (21) mit dem Anschlagselement (23) an dessen Ende ausgebildet ist und bei dem das zylindrische Ventilelement (2) in den zylindrischen Innenraum des Gehäuses (1) eingefügt ist.

7. Hahn nach einem der vorhergehenden Ansprüche, bei dem die Rückhaltevorsprünge (22) eine radial nach innen hervorragende Ausbuchtung in dem Ventilkörper (21) sind.

8. Hahn nach einem der vorhergehenden Ansprüche, bei dem der Vorsprung (12) radial nach außen von der Anschlagseinrichtung (11) hervorragt.

## Revendications

1. Robinet pour le drainage d'un sac de collecte d'urine, comprenant :
un boîtier (1) ayant :
des premier et deuxième passages d'écoulement (101, 102) et un espace interne sensiblement cylindrique et un fond de boîtier ;
un élément de valve rotative (2) qui est monté en rotation dans le boîtier, est doté d'un passage d'écoulement (20), et peut être entraîné en rotation dans le boîtier (1) entre une position de valve fermée et une position de valve ouverte où la communication d'écoulement entre les premier et deuxième passages d'écoulement (101, 102) est prévue ;
l'espace interne du boîtier (1) est doté de premiers moyens de butée de fin de course de rotation (11) qui coopèrent avec des deuxièmes moyens de butée de fin de course de rotation (23) sur l'élément de valve (2) pour limiter le déplacement angulaire de l'élément de valve (2) dans le boîtier (1), **caractérisé en ce qu'**au moins un moyen de butée de fin de course (11) est doté d'une encoche (12) s'étendant radialement à partir du côté externe des moyens de butée de fin de course (11), et **en ce que** l'élément de valve (2) est doté de deux encoches de retenue (22₁, 22₂) s'étendant radialement sur le côté interne du corps de valve cylindrique (21) placé avec un déplacement angulaire inférieur à 90° pour coopérer avec l'encoche (12) des moyens de butée de fin de course (11) du fond de boîtier, de sorte que l'encoche (22₁) fait passer l'encoche (12) dans la position ouverte et que l'encoche (22₂) fait passer l'encoche (12) dans la position fermée juste avant que les premier et deuxième moyens de butée de fin de course viennent en butée entre eux.

2. Robinet selon la revendication 1, dans lequel les premier et deuxième moyens de butée de fin de course de rotation comprennent au moins un moyen de butée de fin de course (11) prévu au fond du boîtier (1) coopérant avec un élément de butée de fin de course (23) prévu sur l'élément de valve (2) pour limiter le déplacement angulaire de l'élément de valve (2) dans le boîtier (1).

3. Robinet selon la revendication 2, dans lequel l'élément de butée de fin de course (23) comprend deux têtes de butée de fin de course (231 ; 232) radialement espacées à 180°.

4. Robinet selon la revendication 2 ou 3, dans lequel deux moyens de butée de fin de course (11) sont prévus avec un déplacement d'approximativement 180° entre eux au fond du boîtier (1).

5. Robinet selon l'une quelconque des revendications 1 à 4, dans lequel les moyens de butée de fin de course (11) des premiers moyens de butée de fin de course de rotation font saillie vers le haut à partir de la surface inférieure dans la direction axiale.

6. Robinet selon l'une quelconque des revendications précédentes, dans lequel l'élément de valve (2) est formé avec un corps de valve (21) sensiblement cylindrique avec l'élément de butée de fin de course (23) au niveau de son extrémité, et en ce que ledit élément de valve (2) cylindrique est inséré dans l'espace interne cylindrique du boîtier (1).

7. Robinet selon l'une quelconque des revendications précédentes, dans lequel les encoches de retenue (22) sont une bosse en saillie radialement vers l'intérieur dans le corps de valve (21).

8. Robinet selon l'une quelconque des revendications précédentes, dans lequel l'encoche (12) fait saillie radialement vers l'extérieur à partir des moyens de butée de fin de course (11).
